# EUROPÄISCHE PATENTSCHRIFT

(11) **EP 3 458 019 B1**
(45) Veröffentlichungstag und Bekanntmachung des Hinweises auf die Patenterteilung: **19.05.2021**
(21) Anmeldenummer: 17729345.3
(22) Anmeldetag: 27.04.2017
(51) Int. Cl.: A61K 8/34, A61K 8/37, A61K 8/39, A61K 8/42, A61K 8/23, A61K 8/67, A61K 8/86, A61Q 5/02, A61Q 7/00, A61Q 19/00, A61Q 19/08, A61Q 19/10, A61K 36/18, A61K 8/9789, A61K 36/185, A61K 36/53, A61K 36/898, A61K 36/899, A61P 17/02, A61P 17/04

(54) **ERZEUGNIS ZUM THERAPIEREN UND VORBEUGEN VON BURNOUT-SYNDROMEN DER KOPFHAUT UND DER HAUT**
PRODUCT FOR THE THERAPY AND PREVENTION OF BURNOUT SYNDROME OF THE SCALP AND THE SKIN
PRODUIT DESTINÉ AU TRAITEMENT ET À LA PRÉVENTION DES SYNDROMES D'ÉPUISEMENT PROFESSIONNEL VISIBLES AU NIVEAU DU CUIR CHEVELU ET DE LA PEAU

(30) Priorität: 18.05.2016 CH 6372016
(43) Veröffentlichungstag der Anmeldung: 27.03.2019
(73) Patentinhaber: APOMEDICA SAGL, 8126 Zumikon (CH)
(72) Erfinder: LUTZ, Beat C., 8125 Zollikerberg (CH)
(74) Vertreter: Felber, Josef
(86) Internationale Anmeldenummer: PCT/CH2017/000039
(87) Internationale Veröffentlichungsnummer: WO 2017/197535

(56) Entgegenhaltungen:
- WO-A1-2017/041834
- CH-B1- 697 723
- CN-A- 104 398 446
- ES-A1- 2 259 274
- JP-A- 2002 226 329
- Trueb: "Was hilft bei empfindlicher Kopfhaut", Ars medici, Bd. 19 31. März 2014 (2014-03-31), Seiten 965-967, XP055387572, Gefunden im Internet: URL:https://www.rosenfluh.ch/media/arsmedi ci/2014/19/Was_hilft_bei_empfindlicher_Kop fhaut.pdf [gefunden am 2017-07-04]
- Anonymous: "CONTRE LE BURNOUT DU CUIR CHEVELU", , 16. April 2016 (2016-04-16), XP055387642, Gefunden im Internet: URL:http://web.archive.org/web/20160416032 625/http://www.hamamelis.ch/documents/Pros pekt_erol_A5_FR_safe.pdf [gefunden am 2017-07-04]
- LITT J Z: "TOPICAL TREATMENT OF ITCHING WITHOUT CORTICOSTEROIDS", ITCH MECHANISMS AND MANAGEMENT OF PRUR, MCGRAW-HILL, NEW YORK, NY, US, 1. Januar 1994 (1994-01-01), Seiten 383-397, XP008054826,
- FREITAG G ET AL: "Results of a postmarketing drug monitoring survey with a polidocanol-urea preparation for dry, itching skin", CURRENT MEDICAL RESEARCH AND OPI, INFORMA HEALTHCARE, GB, Bd. 13, Nr. 9, 1. Januar 1997 (1997-01-01) , Seiten 529-537, XP009123142, ISSN: 0300-7995
- GHONEMY SOHEIR ET AL: "Efficacy and safety of a new 10% topical minoxidil versus 5% topical minoxidil and placebo in the treatment of male androgenetic alopecia: a trichoscopic evaluation", THE JOURNAL OF DERMATOLOGICAL TREATMENT, TAYLOR & FRANCIS, UK, 21 October 2019 (2019-10-21), pages 1-6, XP009523335, ISSN: 1471-1753, DOI: 10.1080/09546634.2019.1654070

## Beschreibung

Empfindliche Kopfhaut führt oft zum sogenannten Red-Scalp-Syndrom und zu einem "Burnout" der Kopfhaut. Klagen über empfindliche Kopfhaut sind ein häufiges Problem in der Praxis und stellen nicht selten eine besondere Herausforderung an den Dermatologen dar. Vor allem Patienten mit Haarausfall und Alopezie (gesteigerter Haarausfall) klagen häufig auch über Probleme der Kopfhaut, die für eine erfolgreiche Therapie im Behandlungsprogramm mit zu berücksichtigen sind. Im Vordergrund steht die Klage über eine empfindliche Kopfhaut, wobei dieser verschiedene Ursachen zugrunde liegen können: Atopische Hautdisposition mit Neigung zu Sebostase, Irritation und Ekzem, Typ-IV-Sensibilisierungen gegenüber bestimmten Shampoo-Inhaltsstoffen (z.B. Cocamidopropylbetain und Konservierungsstoffen), Hauttrockenheit im Alter (Alterssebostase), intensive chemische Haarbehandlungen sowie spezifische dermatologische Kopfhauterkrankungen (z.B. seborrhoisches Ekzem, Psoriasis und ihre therapiebedingten Folgezustände, vornehmlich durch längere Anwendung kortikosteroidhaltiger Wirkstoffe.

Beim Zustand der auf eine übliche antiekzematöse beziehungsweise antiseborrhoische Therapie refraktaren Rötung der Kopfhaut mit Juckreiz und Brennen spricht man wenn nosologisch keine Zuordnung zu einer spezifischen dermatologischen Erkrankung möglich ist, vom Red-Scalp-Syndrom, das heisst die Kopfhaut erscheint durchgehend gerötet und glasig glatt. Einerseits treten die unangenehmen Symptome der empfindlichen Kopfhaut (z.B. Juckreiz, Brennen, Spannungsgefühl) nach dem Auftragen von Haarwuchsmitteln in Erscheinung, wobei sie auf eine austrocknende und irritative Wirkung des in den Lotionen enthaltenen Alkohols und/oder Lösungsmittels Propylenglykol zurückzuführen sind. Andererseits wird das Red-Scalp-Syndrom typischerweise im Bereich der Vertexalopezie beobachtet, die der UV-Strahlung maximal exponiert ist. Pathogenetisch wird es als eine der Rosazea nahestehende, chronische, UV-bedingte Schädigung der Kopfhaut interpretiert. Zudem häufen sich die Hinweise darauf, dass sich die UV-Strahlenexposition neben der Schädigung der Haut auch auf das Haarwachstum negativ auswirkt, vermutlich durch Generierung reaktiver Sauerstoffspezies, wobei gleichzeitig eine erhöhte Empfindlichkeit der Haarpapillenfibroblasten auf oxidativen Stress besteht.

Bei der leicht reizbaren, sensiblen Kopfhaut, die scheinbar nichts mehr verträgt, spricht man auch vom **Burnout-Syndrom der Kopfhaut.** Der Begriff des Burnout bezeichnet allgemein einen Zustand emotionaler Erschöpfung, der als das Ende einer Entwicklungslinie verstanden wird, die mit idealistischer Begeisterung beginnt und über frustrierende Erlebnisse zu Desillusionierung, Depression und Aggressivität führt. Auch beim Zustand der Kopfhaut und bei deren Behandlung mit verschiedenen Therapeutika erlebt der Dermatologe immer wieder ähnliche Patientenkarrieren. Analog zum Bumout-Syndrom, das wissenschaftlich nicht als Krankheit anerkannt ist, sondern gemäss ICD-10 als ein Problem der Lebensbewältigung aufgefasst wird, ist auch das Burnout der Kopfhaut nicht als eigenständige Entität zu verstehen, sondern als Frage der Problembewältigung. Es gilt, die Kopfhaut vor weiteren Insulten der Umwelt, der Medizin und der Kosmetik zu schützen, zu beruhigen und wiederaufzubauen, und das Vertrauen des Patienten wieder zu gewinnen. Aber auch andere Bereiche der Körperhaut können von einem Burn-out-Sydnrom betroffen sein, zum Beispiel die chronisch sonnengereizte Haut, und bedürfen der Behandlung.

Aufgabe der vorliegenden Erfindung ist es deshalb, wirksame Erzeugnisse für die Verhinderung oder Therapierung von Burnout-Syndromen der Haut und insbesondere der Kopfhaut anzugehen.

Die Aufgabe wird gelöst mit einem Erzeugnis zum Vorbeugen und zur Behandlung von Burn-out Symptomen der Haut und insbesondere der Kopfhaut, mit einem Extrakt der Virginischen Zaubernuss (Hamamelis virginiana), Minoxidil (C9H15N50), Rosmarin (Rosmarinus officinalis), Ackerschachtelhalm (Equisetum arvense), Brennnessel (Urticaria dioica), Birken (Betula alba), Salicylsäure (Salcylic Acid), Wasser (Aqua) und Ethanol, als flüssige Haartinktur, die frei von Cocamidopropylbetain und Parabenen ist.

Offenbart ist auch ein Erzeugnis mit einem Extrakt der Virginischen Zaubernuss **(Hamamelis virginiana)** auf einer milden Shampoo-Tensidgrundlage oder als flüssige Haartinktur oder als Creme oder Waschcreme ausgeführt, die frei von Cocamidopropylbetain und Parabenen ist, sowie einen oder mehrere Zusatzstoffe aus folgender Auswahl enthält:
- Tocopherol
- Rosmarin (Rosmarinus officinatis)
- Urea
- Laureth 9
- Zea Mays Corn Kernel Extract
- Glycerin
- Isopropyl Myristate
- Polysorbate 80
- Sulfur (Bioschwefel).

Ungeachtet eines angewendeten Erzeugnisses bildet bei einem Burn-out Syndrom der Kopfhaut die Optimierung der Haarwaschfrequenz den ersten Schritt in der Behandlung und der Prävention der gereizten Kopfhaut. Menschen mit fettigen Haaren sollten oft, mitunter auch täglich, Menschen mit trockenen Haaren weniger oft ihre Haare waschen. Zudem ist auf ein mildes Shampoo zu achten, wobei Inhaltsstoffe, die ein hohes Irritationspotenzial aufweisen oder ein bekanntes Kontaktallergen enthalten, zu vermeiden sind.

Von besonderem Interesse im idealen Erzeugnis zum Therapieren des Kopfhaut-Burnout sind reizlindernde Wirkstoffe aus der traditionellen Phytomedizin, vorallem und insbesondere die Virginische Zaubernuss **(Hamamelis virginiana),** sowie zusätzlich oder ohne die nachfolgenden pflanzlichen Inhaltsstoffe wahlweise Kamille (Marticaria chamornilla), Herzsamen (Cardiospermum halicacabum), Pfingstrose (Paeonia lactiflora).

Als die Europäer den amerikanischen Ureinwohnern erstmals begegneten, verwendeten die Stämme im Nordosten Amerikas etwa 275 Pflanzen zu medizinischen Zwecken. **Hamamelis virginiana** zählte zu den wichtigsten unter ihnen (Behandlung oberflächlicher Hautverletzungen und Hautentzündungen). Ab Mitte des 19. Jahrhunderts wurde Hamamelis schliesslich Bestandteil der offiziellen Medizin in Europa und Nordamerika. Verwendet werden die Laubblätter (Hamamelidis folium) sowie die Rinde (Hamamelidis cortex) der in Nordamerika heimischen Virginischen Zaubemuss. Vom Missionar Dr. Charles Hawes entdeckt, hat das Destillat der Heilpflanze Hamamelidis ab 1866 von der nordamerikanischen Ostküste aus seinen Siegeszug in die dermatologischen Praxen der Welt gehalten und heute mehr denn je an Aktualität gewonnen.

Heute werden aus Hamamelis verschiedene Spezialitäten hergestellt und wirtschaftlich verwendet, jedoch bisher keines spezifisch für die Behandlung der Kopfhaut und der Haut mit einem sogenannten Burnout oder einem Red-Scalp-Syndrom, Red-Skin-Syndrom oder gegen das Burnout sonnengereizter Haut und Kopfhaut. Von entscheidender Bedeutung für die Wirksamkeit eines solchen Erzeugnisses ist die Qualität des eingesetzten **Hamamelis virginiana.** Gewöhnliches Hamamelisdestillat (HMM-Wasser) nämlich weist nur geringe Wirkstoffpeaks auf. Um eine rasche Regenerierung der Kopfhaut mit positivem Einfluss auf den übrigen Therapieverlauf zu erreichen, ist auf hochwertige Hamameliswirkstoffe aus wildwachsender und nachhaltiger Produktion zu achten. Ein besonders wirksames Erzeugnis, speziell zur Pflege und zur Behandlung der empfindlichen Kopfhaut und Haut, besteht aus einem Extrakt der Virginischen Zaubernuss (Hamamelis virginiana) auf einer milden Shampoo-Tensidgrundlage, die frei von Cocamidopropylbetain und Parabenen ist, oder einer anderen milden dermatologischen Grundlage ohne allergene Inhaltsstoffe.

In der Dermatologischen Klinik des Universitätsspitals Zürich wurde das neuentwickele Shampoo-Präparat und zusätzlich auch das Erzeugnis in Form eines Hair Tonic in einem Zeitraum von 6 Monaten im Rahmen einer Anwendungsbeobachtung an Patienten abgegeben, die über subjektive Reizerscheinungen der Kopfhaut klagten (mit oder ohne objektivierbare Veränderungen wie Rötung, Schuppung, Papeln und Exkoriationen). Viele Patienten hatten zuvor bereits ohne Erfolg verschiedene Medizinalshampoos, vornehmlich gegen Seborrhö und Schuppen, sowie kortikosteroidhaltige Externa eingesetzt. Die Mehrzahl der Patienten berichtete nach einer Anwendungszeit von 4 Wochen über eine Besserung der subjektiven Reizerscheinungen, wobei sie nach der Anwendung beide Präparate als gleich gut bis ausgezeichnet verträglich einstuften. Insgesamt war die Mehrzahl der Patienten mit den Produkten zufrieden. Die dokumentierte Wirksamkeit ist vornehmlich auf **Hamamelis virginiana** zurückzuführen. Hauptwirkstoffe sind die in Hamamelis enthaltenen Flavonoide und Polyphenole, die eine natürliche Quelle von Antioxidanzien und Radikalfängern darstellen. Die im entwickelten Präparat Haar Tonic zusätzlich enthaltenen pflanzlichen Inhaltsstoffe fördern den Stoffwechsel und die Durchblutung (Rosmarin, Acker-Schachtelhalm), hemmen das Wachstum von Propionibakterien (Rosmarin - Rosmarinus officinalis), sind gegen Schuppen wirksam (Rosmarin - Rosmarinus officinalis), stärken das Bindegewebe (Acker-Schachtelhalm) und regen das gesunde Haarwachstum an (Birke, Brennnessel).

Die Untersuchungen wurden an 1373 Patienten (1233 Frauen und 140 Männer) zur Behandlung der reizbaren Kopfhaut oder als Begleitbehandlung einer Minoxidil-Therapie bei androgenetischer Alopezie durchgeführt. 369 (26,9%) bezogen das neue Erzeugnis in diesem Zeitraum mehr als einmal. Die Lokalanwendung topischer Minoxidil-Produkte (2% bis 10% Wirkstoffanteil) in einer alkoholisch-wässrigen Grundlage zur Behandlung der androgenetischen Alopezie führte erfahrungsgemäss häufig zu Irritatioriserscheinungen der Kopfhaut, die sich negativ auf die Patientencompliance auswirken konnte. Vielfach werden die Beschwerden der Patienten und die Rötung der Kopfhaut als seborrhoisches Ekzem fehlinterpretiert und mit irritierenden Antischuppen-Shampoos oder topischen Kortikosteroiden in alkoholischer Grundlage (Scalp Applications) behandelt, was die Problematik nur weiter zuspitzt. Als Begleittherapie zur topischen Minoxidil-Therapie bei androgenetischer Alopezie eignet sich jedoch die qualitativ hochwertige, reizarme Shampoobehandlung auf der Basis von Hamamelis virginiana aus Wildbeständen.

Fazit für die Praxis: Die Wahl geeigneter Haarpflegeprodukte in Form eines geeigneten Shampoos und eines ebenso geeigneten Haar Tonics stellt einen wichtigen Aspekt im Behandlungsplan der empfindlichen Kopfhaut dar. Bei der Haarwäsche als häufigste Form der Kopfhaut- und Haarbehandlung hat das Shampoo mehr zu leisten als nur zu reinigen. Es sollte auch auf die Bedürfnisse der verschiedenen Haarqualitäten, das Alter und die individuellen Waschgewohnheiten zugeschnitten sein. Besonders sollte es die Probleme der Kopfhaut rasch positiv beeinflussen. Mit dem im Anspruch 1 definiertem Erzeugnis stehen die Vorzüge von Hamamelis nun auch für die Behandlung der Kopfhaut zur Verfügung, insbesondere im Zusammenhang mit der Problematik des Red-Scalp-Syndroms, des Burnouts der Kopfhaut und der Haut und der topischen Minoxidil-Therapie bei androgenetischer Alopezie.

Offenbart, aber nicht Teil der Erfindung, ist eine Creme. Als Crème für die Haut enthält diese nebst den wichtigen Wirkstoffen **Hamamelis virginiana** noch folgende weitere, wesentliche hautpflegende Inhaltsstoffe: **Harnstoff, Panthenol und Tocopherol.** Diese Stoffe pflegen die Haut und schützen sie vor den zunehmend schädlichen Belastungen durch unsere Umwelt. Die genannten hautpflegenden Inhaltsstoffe der Crème steigern die Wasserbindungsfähigkeit, die Mikrozirkulation und den Nährstofftransport der Haut und sorgen auf diese Weise für ein frischeres Hautbild. Im Einzelnen:

**Hamamelisextrakt:** Der in den Spezialitäten verwendete Hamamelisextrakt aus Wildbeständen von Hamamelis virginiana enthält die pflanzlichen Stoffgruppen der Kohlenwasserstoffe, Isoprenoide und Phenylpropane mit ausgeprägten antioxidativen und hautschützenden Eigenschaften. In verschiedenen humanklinischen Untersuchungen gewonnene analytische Daten belegen diese positive Wirkung.

**Urea (Harnstoff):** Urea (Harnstoff) dient der Erhöhung des Wasserbindevermögens der Haut. Harnstoff entsteht natürlicherweise in unserem Körper als Endprodukt des Aminosäurenstoffwechsels. Die Zufuhr von Harnstoff in bestimmten Dosen stabilisiert die Struktur der Proteine durch die Erhöhung des Wasserbindevermögens.

**Panthenol:** Panthenol wird in unserem Körper zu Pantothensäure umgewandelt, die ein Bestandteil des Coenzyms A ist und eine wesentliche Rolle im Hautstoffwechsel spielt. Panthenol wird in Öl-Wasser-Emulsionen (Crème) verarbeitet und wird von der Haut gut aufgenommen und reichert sich am Anwendungsort an. Es erhöht das Feuchthalte-vermögen der Haut, hat somit pflegende Eigenschaften, und verbessert ihre Elastizität. Panthenol fördert die Neubildung der Hautzellen und trägt auf diese Weise zu ihrer Regeneration bei. Darüber hinaus hat Panthenol auch juckreizlindernde, entzündungs-hemmende und wundheilungsfördernde Eigenschaften.

**Tocopherol:** Bei Tocopherol handelt es sich um die fettlösliche Form von Vitamin E, das aufgrund seiner stark antioxidativen Eigenschaften die Zellwände gegen freie Radikale schützt. Zudem fördert Tocopherol den Kollagengehalt in der Haut, wodurch sie straffer wird. Wunden heilen schneller, da die Bildung neuer Zellen nicht durch freie Radikale gestört wird. Auch schützt Tocopherol vor Hautschäden durch übermässige Sonnen-einstrahlung. Insgesamt verleiht Vitamin E der Haut ein jüngeres und frischeres Aussehen.

Eine Crème mit all diesen Inhaltsstoffen eröffnet ein weiteres Spektrum von dermatologischen Anwendungen, die im Folgenden erwähnt und erläutert werden, wobei die Daten und Aussagen sich auf Anwendung der Crème an Patienten stützen. Die Ergebnisse der Anwendungen wurden dort auch protokolliert und ausgewertet. Die Crème ist eine kosmetische Lotion zur dermatologischen Hautpflege. Sie wird schnell von der Haut aufgenommen und ist sehr gut verträglich. Darüber hinaus ist die Crème auch in der Lage, leichtere dermatologische Probleme wie eine Entzündung, Rötung, Juckreiz sowie trockene und raue Haut positiv zu beeinflussen. Die bei den genannten Beschwerden festgestellten Wirkungen dürften in erster Linie auf das in der Crème enthaltene **Hamamelis virginiana** zurückzuführen sein. Hamamelis-Wirkstoffe sind aufgrund ihrer entzündungshemmenden und antioxidativen Eigenschaften in einer Vielzahl kosmetischer und arzneilicher Zubereitungen zu finden und kommen sowohl in der Hautpflege als auch bei der Behandlung von Hautkrankheiten zum Einsatz. Dabei kann die Anwendung geeigneter Hamamelispräparate zum Teil die Verwendung nebenwirkungsreicher Kortikoide ersetzen. Im Einzelnen wurden folgende Sachverhalte nach der Anwendung der Crème bei verschiedenen Symptomen festgestellt:

**Bei leichteren Verbrennungen** (Referenzbeispiel): Die Crème war bei 10 behandelten Patienten in der Lage, durch Sonnenbrand hervorgerufene Beschwerden zu lindern. Die beobachteten Patienten litten unter den Folgen eines Sonnenbrandes 1. Grades mit Rötungen, leichten Schwellungen, Schmerzen und Juckreiz. Die Creme wurde dabei über einige Tage hinweg mehrmals täglich angewendet. Beobachtet werden konnte ein rascher Rückgang der Beschwerden (Abklingen der Rötungen und Schwellungen, Nachlassen von Schmerz und Juckreiz). Leichte und kleinflächige Verbrennungen wie zum Beispiel im Gesicht oder an den Armen waren bereits am nächsten Tag ausgeheilt.

**Bei trockener** Haut (Referenzbeispiel): Diabetiker leiden oft unter trockener Haut, insbesondere an den Füssen. Aus diesem Grund werden ihnen vom Facharzt Haut befeuchtend wirkende Präparate verordnet. Die Crème wurde von 13 beobachteten Patienten mit leichter bis mittelgradig trockener Haut 2x täglich leicht in die Füsse einmassiert und hatte bei 11 Patienten den gleichen Nutzen wie die befeuchtenden Spezial-Präparate mit hohem Harnstoffgehalt. Einige dieser Patienten litten auch unter trockenen und rauen Händen und lobten den positiven Einfluss der Lotion auf ihre Hände, speziell in der kalten Jahreszeit. Der Beobachtungszeitraum erstreckte sich über 3 Monate.

**Bei** Stichverletzungen (Referenzbeispiel): Bei kleineren Stichverletzungen insulinpflichtiger Diabetiker führte die regelmässige Anwendung der Crème zu einer rascheren Wundheilung mit verringerter Narbenbildung. Insulinpflichtige Diabetiker müssen täglich mehrmals ihren Blutzucker messen, wobei durch die erforderliche Entnahme einer geringen Blutmenge jedes Mal eine kleine Stichverletzung entsteht, vor allem an den Seiten der Fingerkuppen. Der günstige Effekt auf die Wundheilung konnte bei allen 15 behandelten und beobachteten Patienten festgestellt werden. Der Anwendungszeitraum erstreckte sich über 4 Wochen, wobei die Hamamelis-Spezialität 2x täglich auf die Wundstellen aufgetragen wurde.

**Bei** Ekzemen (Referenzbeispiel): Beschwerden wie Juckreiz und Rötung, die bei Patienten mit den ekzematösen Hauterkrankungen Neurodermitis und Psoriasis auftraten, konnten in den meisten Fällen zumindest etwas gelindert werden. Beobachtet wurden insgesamt 8 Patienten, welche die Creme 2x täglich in einem Zeitraum von 4 Wochen anwendeten. Bei 3 Patienten konnte eine deutliche, bei 2 weiteren eine leichte Verbesserung der Beschwerden Juckreiz und Rötung festgestellt werden. 2 Patienten mit Neurodermitis brachen die Behandlung ab, da sich die Beschwerden eher etwas verschlimmerten. Langzeit-Neurodermitiker reagieren oft empfindlich auf eine Vielzahl von Stoffen, auch wenn diese natürlichen Ursprungs sind. Bei einem einzigen Psoriasispatient zeigte sich zwar keine Besserung von Juckreiz und Rötung, aber eine Minderung der Hauttrockenheit für die Dauer der Anwendung.

**Beim Red Skin** Syndrom (Referenzbeispiel): Zum Red Skin Syndrom mit oft ausgeprägter Rötung und Juckreiz kommt es meist nach der langfristigen, oft hoch dosierten topischen (lokalen) Anwendung von Kortikosteroiden, wie sie in der Behandlung entzündlicher Ekzeme beispielsweise bei Psoriasis und Neurodermitis erfolgt In einem beobachteten Fall des Red Skin Syndroms war die Crème in der Lage, den Juckreiz und die Rötung deutlich und rasch zu lindern, in einem weiteren Behandlungsfall kam es zu einer leichten Linderung. Die Creme wurde 4 Wochen lang 2x täglich angewendet

Für die in den Praxiserfahrungen festgestellten entzündungshemmenden und juckreiz-lindernden Eigenschaften der Crème ist mit grösster Wahrscheinlichkeit vor allem der Hauptwirkstoff Hamamelis virginiana in einer dermatologisch angepassten Zusammensetzung verantwortlich. Aus diesem Grund folgt an dieser Stelle noch einmal eine ausführlichere Beschreibung von Hamamelis.

Die aus Nordamerika stammende Hamamelis virginiana gilt bei den Indianern Nord-amerikas traditionsgemäß als wertvolle Heilpflanze. Seit vielen Jahrhunderten verwenden die amerikanischen Ureinwohner wässrige Zubereitungen von Blättern und Rinde der virginischen Zaubernuss bei Hautentzündungen aller Art wie zum Beispiel juckenden Ekzemen, bei Wunden und Verletzungen, Geschwüren, Krampfadern, Hämorrhoiden und Durchfall. Seit Mitte des 19. Jahrhunderts ist Hamamelis auch Bestandteil der offiziellen Medizin Europas und Nordamerikas. Die Sachverständigenkommission des Bundesgesundheitsamtes nennt für getrocknete Hamamelisblätter und Hamamelisrinde folgendeAnwendungsgebiete:
- Leichte Hautverletzungen,
- lokale Entzündungen von Haut und Schleimhäuten,
- Krampfaderbeschwerden,
- Unterstützung der Therapie akuter unspezifischer Durchfallerkrankungen,
- Entzündungen von Zahnfleisch und Mundschleimhaut.

Das Testergebnis der Hamamelisqualität in der getesteten Creme belegte eindeutig die entzündungs-hemmende Wirkung, durchgeführt an menschlichen Lymphozyten durch das Labor für chemische Immunologie am Krankenhaus für übertragbare Krankheiten in Polen. Die wesentlichen Inhaltsstoffe von Hamamelis virginiana sind Flavonoide und Tannine (Polyphenole). Sie unterstützen das Immunsystem bei der Bekämpfung von Krankheitserregern und der Neutralisierung von Giftstoffen. Flavonoide vermindern aggressive freie Radikale und neutralisieren schädliche Enzyme, die die wesentlichen Hautbestandteile wie Kollagen, Elastin und Hyaluronsäure (langkettige Polysaccharide) zerstören. Bei freien Radikalen handelt es sich um sehr reaktive Sauerstoffmoleküle, die, sobald sie im Übermass vorhanden sind, die Hautzellen schädigen und den Alterungsprozess beschleunigen. Dabei kommt es zu Schäden an einzelnen Zellbestandteilen wie Enzymen, Eiweissen und Lipiden, zu Schäden an den Zellmembranen wie auch an den Erbanlagen im Zellkern. Antioxidative Wirkstoffe sind aus diesem Grund sehr willkommen und geeignet, Hautschäden vorzubeugen. Sie fördern die Regeneration der Haut bei dermatologischen Krankheiten und verlangsamen den Alterungsprozess. Polyphenole kann man daher auch als Anti-Aging Wirkstoffe bezeichnen.

## Patentansprüche

1. Erzeugnis zum Vorbeugen und zur Behandlung von Burn-out Symptomen der Kopfhaut, mit einem Extrakt der Virginischen Zaubernuss **(Hamamelis virginiana),** Minoxidil (C9H15N5O), Rosmarin (Rosmarinus officinalis), Ackerschachtelhalm (Equisetum arvense), Brennnessel (Urticaria dioica), Birken (Betula alba), Salicylsäure (Salicylic acid), Wasser (Aqua) und Ethanol, als flüssige Haartinktur, die frei von Cocamidopropylbetain und Parabenen ist.

## Claims

1. Product for the prevention and treatment of burnout symptoms of the scalp, with an extract of Virgin Witch Hazel **(Hamamelis virginiana),** Minoxidil (C9H15N5O), Rosemary (Rosmarinus officinalis), Field Horsetail (Equisetum arvense), Stinging Nettle (Urticaria dioica), Birch (Betula alba), Salicylic Acid (Salcylic Acid), Water (Aqua) and Ethanol, as a liquid hair tincture free of Cocamidopropyl Betaine and Parabens.

## Revendications

1. Produit pour la prévention et le traitement des symptômes d'épuisement du cuir chevelu, avec un extrait d'hamamélis vierge **(Hamamelis virginiana),** de minoxidil (C9H15N5O), de romarin (Rosmarinus officinalis), Prêle des champs (Equisetum arvense), ortie dioïque (Urticaria dioica), bouleau (Betula alba), acide salicylique (Salcylic Acid), eau (Aqua) et éthanol, sous forme de teinture capillaire liquide exempte de cocamidopropyl bétaïne et de parabènes.
